(19) Europäisches Patentamt / European Patent Office / Office européen des brevets

(11) **EP 3 639 276 B1**

(12) **EUROPEAN PATENT SPECIFICATION**

(45) Date of publication and mention
of the grant of the patent:
**07.05.2025 Bulletin 2025/19**

(21) Application number: **18734116.9**

(22) Date of filing: **12.06.2018**

(51) International Patent Classification (IPC):
*G16H 50/30* (2018.01)

(52) Cooperative Patent Classification (CPC):
**G16H 50/30**

(86) International application number:
**PCT/EP2018/065412**

(87) International publication number:
**WO 2018/229019 (20.12.2018 Gazette 2018/51)**

(54) **RISK ASSESSMENT OF DISSEMINATED INTRAVASCULAR COAGULATION**

RISIKOBEWERTUNG VON DISSEMINIERTER INTRAVASKULÄRER KOAGULATION

ÉVALUATION DU RISQUE DE COAGULATION INTRAVASCULAIRE DISSÉMINÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.06.2017 US 201762518064 P**

(43) Date of publication of application:
**22.04.2020 Bulletin 2020/17**

(73) Proprietor: **Koninklijke Philips N.V.
5656 AG Eindhoven (NL)**

(72) Inventors:
• **MARGARITO, Jenny
5656 AE Eindhoven (NL)**
• **BAKKER, Bart, Jacob
5656 AE Eindhoven (NL)**
• **VAN DEN HAM, Ren
5656 AE Eindhoven (NL)**

(74) Representative: **Philips Intellectual Property & Standards
High Tech Campus 52
5656 AG Eindhoven (NL)**

(56) References cited:
**WO-A2-2009/123737     US-A1- 2004 097 460**

• **KANG Y. R ET AL: "Initial lactate level and mortality in septic shock patients with hepatic dysfunction", ANAESTHESIA AND INTENSIVE CARE, 1 January 2011 (2011-01-01), Edgecliff, pages 862 - 867, XP055840012, Retrieved from the Internet <URL:https://journals.sagepub.com/doi/pdf/10.1177/0310057X1103900510> [retrieved on 20210910], DOI: 10.1177/0310057X1103900510**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Description

FIELD OF THE INVENTION

The present invention relates to a method and system for the assessment of the risk of the development of disseminated intravascular coagulation (DIC), in patients showing systemic inflammatory response syndrome (SIRS), or sepsis.

BACKGROUND OF THE INVENTION

DIC is a pathological process characterized by a systemic activation of the blood coagulation system, leading to subsequent clot formation, blood vessel obstruction and organ dysfunction. The large consumption of platelets and coagulation factors in this process may in turn cause bleeding, which further worsens the patient's condition and decreases the chances of survival (Di Nisio, M., et al., "Diagnosis and treatment of disseminated intravascular coagulation: guidelines of the Italian Society for Haemostasis and Thrombosis (SISET)," Thrombosis research, vol. 129, pp. e177-e184, 2012.)

DIC is usually secondary to an underlying condition such as systemic inflammatory response syndrome (SIRS), sepsis, trauma, malignancy, heat stroke and hyperthermia. SIRS and sepsis are among the most common causes of DIC, whose mortality ranges between 10-50%. Between 30% and 50% of sepsis patients develop DIC. Sepsis severity positively correlates with DIC incidence and therefore mortality (Levi. M., et al., "Disseminated intravascular coagulation," New England Journal of Medicine, vol. 341, pp. 586-592, 1999). DIC incidence ranges between 7% (mild sepsis) and 73% (septic shock) (see Kinasewitz, G.T., et al., "Prognostic value of a simple evolving disseminated intravascular coagulation score in patients with severe sepsis," Critical Care Medicine, vol. 33, pp. 2214-2221, 2005), and DIC mortality ranges between 10% and 50%.

Several diagnostic scores based on general coagulation tests such as prothrombin time (PT), fibrinogen, d-dimer and platelet counts have been proposed to objectify the subjective clinical diagnosis of DIC based on the clinical signs and symptoms of the patient. See, e.g., Kobayashi, N., et al., "Criteria for diagnosis of DIC based on the analysis of clinical and laboratory findings in 345 DIC patients collected by the Research Committee on DIC in Japan," Disseminated Intravascular Coagulation, ed: Karger Publishers, 1983, pp. 265-275. Although some of those scores seem to reflect the clinical diagnosis of DIC by experts quite well, this also means that these scores are unsuitable as a tool for the risk assessment and prevention of DIC.

WO 2009/123737 discloses methods, monitors and systems for advanced detection of sepsis in a subject, but it is silent about the detection of disseminated intravascular coagulation.

SUMMARY OF THE INVENTION

The invention is defined by the independent claims. The dependent claims define advantageous embodiments. Embodiments of the invention provide a computer program product for implementing a method for early DIC assessment and preventive treatment planning, which has the potential for significantly decreasing mortality rate as well as the rate of DIC related sequelae in the SIRS/sepsis patient population and thereby improving quality of life. The risk assessment method is based on features of vital signs and/or biomarker measurements, and provides solutions for assessing the risk of DIC development 24 hours in advance or within 72 hours after ICU admittance. Optimal sets of measurements for each model implementation are selected. The invention also provides a computer readable storage medium encoded with the computer-implementable instructions of the computer program product, and a system having a processor programmed with the computer program product.

Developed models obtaining an area under the ROC (receiving operation curve) curve (AUC) equal to 0.85 and 0.83, for the risk assessment 24 hours in advance and within 72 hours from ICU admittance, are described.

The system provides an improved process of integrative analysis of a patient's characteristics and demographics, vital signs and in-vitro diagnostic data for effective treatment planning.

The high mortality of SIRS/sepsis associated DIC has driven us to develop methods for assessing the risk of DIC development following the diagnosis of SIRS, using both vital signs and standardly available laboratory measurements. These methods allow for improving the prevention of DIC in SIRS patients, which in turn improves patient outcomes.

This invention assesses the risk of the development of DIC in SIRS patients. While available diagnostic DIC scores only allow for therapeutic treatment of DIC, the within invention allows for early and preventative intervention that should decrease DIC incidence and reduce mortality. Currently no risk assessment algorithms are available for DIC despite its high mortality rate.

Accordingly, the invention makes use of a method based on biomarkers (lactate, bilirubin and creatinine) and/or statistical features (e.g., average, standard deviation, kurtosis, skewness and quantile values) extracted from vital signs (heart rate, respiration rate and oxygen saturation) that assesses the risk of developing DIC 24 hours in advance. In addition, the invention makes use of a method based on biomarkers (lactate, bilirubin and creatinine) and/or statistical features (e.g., average, standard deviation, kurtosis, skewness and quantile values) extracted from vital signs (heart rate, respiration rate and oxygen saturation), that assesses the risk of developing DIC within the first 72 hours after ICU admittance.

The optimal system requires all mentioned biomarker measurements on at least a daily basis and continuous monitoring of all mentioned vital signs. More practical, but still high performing implementations are based on continuous screening of sepsis/SIRS patients using one or more of the vital signs (high sensitivity, low specificity) and subsequent measurement of one or more of the biomarkers to increase the specificity of the risk assessment at the moment the vital signs indicate an increased risk of DIC development.

In general terms, our invention uses the combination of vital signs monitoring for the detection of clinical deterioration of sepsis/SIRS patients, with biomarker measurements that reflect organ damage likely caused by the DIC. One skilled in the art could imagine solutions that combine vital signs monitoring data with other and/or earlier markers of organ damage, and/or more direct biomarkers of systemic activation of the coagulation system. It is an object of the present invention to provide a computer-implemented method and system for assessing the risk of development of DIC in patients showing SIRS or sepsis. In particular, it is an object of the present invention to provide a system and method that solves the above-mentioned problems of the prior art by providing a method and system for assessing the risk of DIC development. This invention proposes two different implementations of the system, the first one allows for predicting the onset of DIC 24 hours in advance, while the second one allows for predicting the risk of DIC at ICU admittance, more specifically in the first 72 hours from ICU admittance, before DIC is being diagnosed.

It is also an object of the present invention to provide a system and method for assessing the risk of developing DIC based on features of vital signs and/or biomarker measurements. It is a further object of the present invention to provide a method and system for decreasing the mortality rate for patients developing DIC by early assessment and preventative treatment planning for DIC. It is also an object of the present invention to provide an alternative to the prior art.

Thus, the above-described object and several other objects are intended to be obtained in a first aspect of the invention by providing a system and method for providing relevant patient-specific DIC risk information, such system and method comprising:

diagnosing said patient for SIRS and, if positive, inputting the patient-specific diagnostic data onto a processor configured to receive said patient-specific data, and storing said data on a non-transitory computer readable storage medium;

acquiring initial vital signs and biomarker measurement data from said patient and inputting and storing said vital signs and biomarker measurement data on said non-transitory computer readable storage medium;

monitoring said vital signs of said patient and continuously inputting and storing said vital signs and biomarker measurement data on said non-transitory computer readable storage medium;

pre-processing said vital sign data, including assessing the quality of said data and removing outliers from said data (the pre-processing consists of discarding samples which are outside the physiological range; physiological range for both biomarkers and vital signs are shown in Table 1 and Table 2 below);

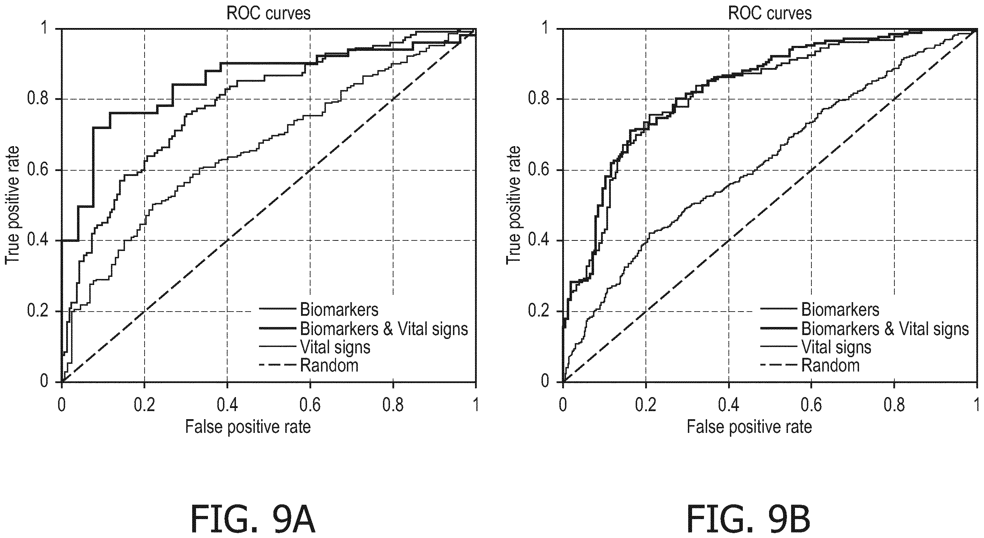

windowing of said vital signs data; vital signs are segmented in sliding windows 120 minutes long with an overlap of 60 minutes. The statistic of interest is extracted from each window and the final feature is obtained by averaging the statistics extracted over all the windows (see FIG 9).

extracting specific features from said patient-specific data;

calculation of statistical features from said vital signs windows (a description of the statistical features is provided in Table 3 below);

analyzing said patient data with respect to a predictive model that is stored on said non-transitory computer readable storage medium; and

determining whether a value derived from said patient-specific data by the predictive model meets a DIC probability threshold, wherein said probability threshold is predictive of the likely development of DIC.

Table 1 Physiological range for selected biomarkers

| Name | Unit Measure | Range |
|---|---|---|
| creatinine | mg/dl | 0 ÷ 20 |
| direct bilirubin | mg/dl | 0 ÷ 10 |
| lactate | mmol/L | 0 ÷ 10 |

(continued)

| Name | Unit Measure | Range |
|---|---|---|
| total bilirubin | mg/dl | 0 ÷ 20 |

Table 2 Physiological range for selected vital signs

| Name | Unit Measure | Range |
|---|---|---|
| Oxygen saturation | % | >= 90 |
| Heart Rate | bpm | 25 ÷ 250 |
| Respiration | Cycle/min | > 0 |

Table 3 Description of statistical features extracted from vital signs

| Name of the features | Description |
|---|---|
| Mean | average value of the signal point values |
| Standard deviation | root Mean squared deviation of the signal point values from their arrhythmic mean |
| Kurtosis | it quantifies the sharpness of the distribution curve peak |
| Skewness | it quantifies asymmetry of a distribution around its mean |
| Quantile 0.25 | x-value of the distribution which includes 0.25*N observations, with N being the number of point values |
| Quantile 0.50 | x-value of the distribution which includes 0.50*N observations, with N being the number of point values |
| Quantile 0.75 | x-value of the distribution which includes 0.75*N observations, with N being the number of point values |
| Range | Difference between the maximum and the minimum signal point values for a given signal |
| Energy | sum of the squared signal point values |

## BRIEF DESCRIPTION OF THE DRAWINGS

The methods and system according to the invention will now be described in more detail with regard to the accompanying figures. The figures show ways of implementing the present invention and are not to be construed as being limiting to other possible embodiments falling within the scope of the attached claims.

FIG. 1 illustrates biomarker distributions for patient populations who developed (Case) and did not develop DIC (Control);

FIG. 2A is a plot showing the area under the curve (AUC) for single biomarkers, subject characteristics, the Apache score and statistic features extracted from vital signs for the 24-hour model;

FIG. 2B is a plot showing the area under the curve (AUC) for single biomarkers, subject characteristics, the Apache score and statistic features extracted from vital signs for the 72 ICU admittance hours model;

FIG. 3 is a diagram illustrating the three versions of the DIC risk assessment framework;

FIG. 4 is a block diagram showing an embodiment of the DIC risk assessment system of the within invention;

FIG. 5 is a flowchart of an embodiment of the invention showing a process from patient ICU admission and diagnosis leading to risk assessment for DIC;

FIG. 6 is a flowchart describing the pre-processing steps that precede training of logistic functions which compose a final risk assessment model;

FIG. 7 is a diagram of an implemented model of the within invention; the parameter q and w were determined during the model training;

FIG. 8 provides two flowcharts describing steps for estimating the DIC probability giving a generic vital sign or a generic biomarker. The parameters **k** and **m** were determined with the training of the model;

FIG. 9A is the AUC calculated on the test set for the different configurations for the 24-hour model;

FIG. 9B is the AUC calculated on the test set for the different configurations for 72 ICU admittance hours model;

FIG. 10 is the representation of the logistic function applied for calculating the probability of DIC occurrence.

## DETAILED DESCRIPTION OF THE INVENTION

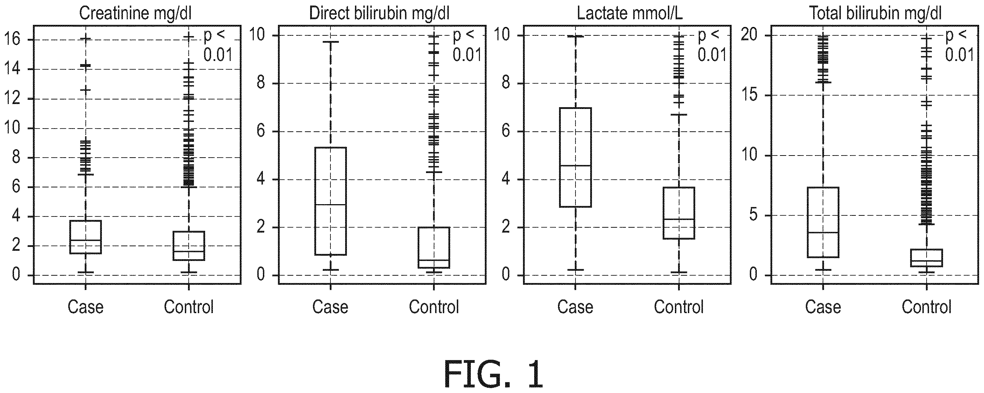

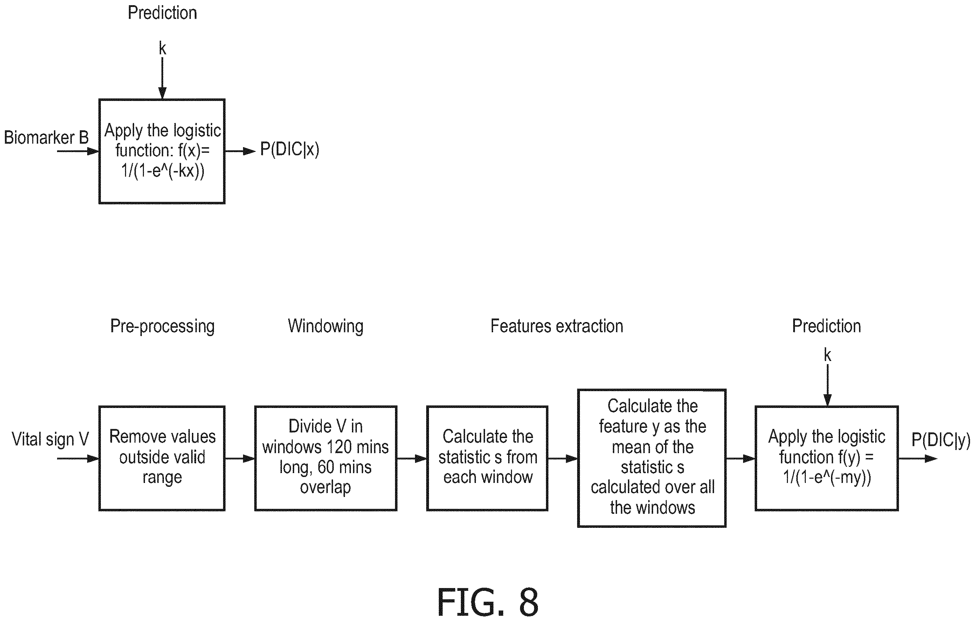

The present invention provides a method and system for early risk assessment of DIC using biomarker measurements, vital signs monitoring and the combination of vital signs monitoring for the detection of clinical deterioration of sepsis/SIRS patients, with biomarker measurements that reflect organ damage likely caused by the DIC. The present invention is described in further detail below with reference made to FIGS. 1-10.

This invention arises from the analysis of intensive care unit (ICU) data collected from SIRS patients that did not receive anti-coagulant treatment before DIC diagnosis. The selected patients were divided into training and test sets, 70% and 30%, respectively. The training set has been used for determining the model parameters, whereas the test set to evaluate the model performance. Descriptions of the training and test sets are provided in Table 4. Data from patient subgroups have been used to develop the models of this invention since not all data was always available for all selected SIRS patients.

**Table 4 Patients sets**

|  | Model 24 hours | | Model 72 hours ICU | |
|---|---|---|---|---|
|  | **Training set** | **Test set** | **Training set** | **Test set** |
| **Nr. Subjects** | 2277 | 947 | 2652 | 1120 |
| **Nr. Cases** | 1082 | 455 | 1557 | 670 |
| **Nr. Controls** | 1195 | 492 | 1095 | 450 |
| **Male [%]** | 54 | 51 | 54 | 52 |
| **Age (sd) [years]** | 62 (16) | 61 (16) | 61 (16) | 60 (16) |
| **Weight (sd) [kg]** | 78.3 (21.0) | 78.4 (21.5) | 78.1 (21.0) | 78.2 (21.0) |
| **Height (sd) [m]** | 1.69 (0.12) | 1.69 (0.11) | 1.69(0.12) | 1.69 (0.11) |
| **BMI (sd) [kg/$m^2$]** | 27.2 (6.1) | 27.4 (6.4) | 27.2(6.2) | 27.3 (6.3) |

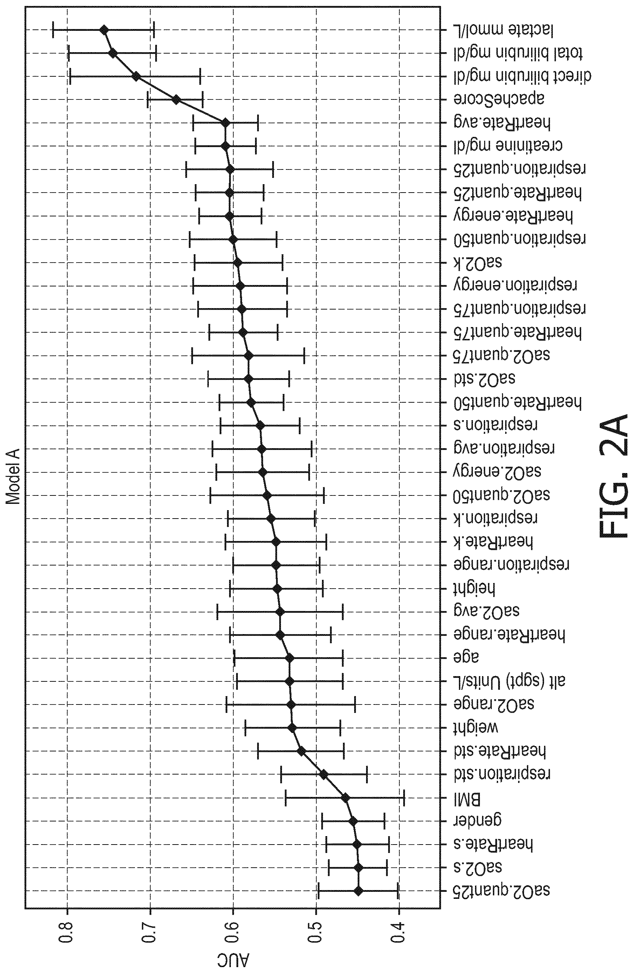

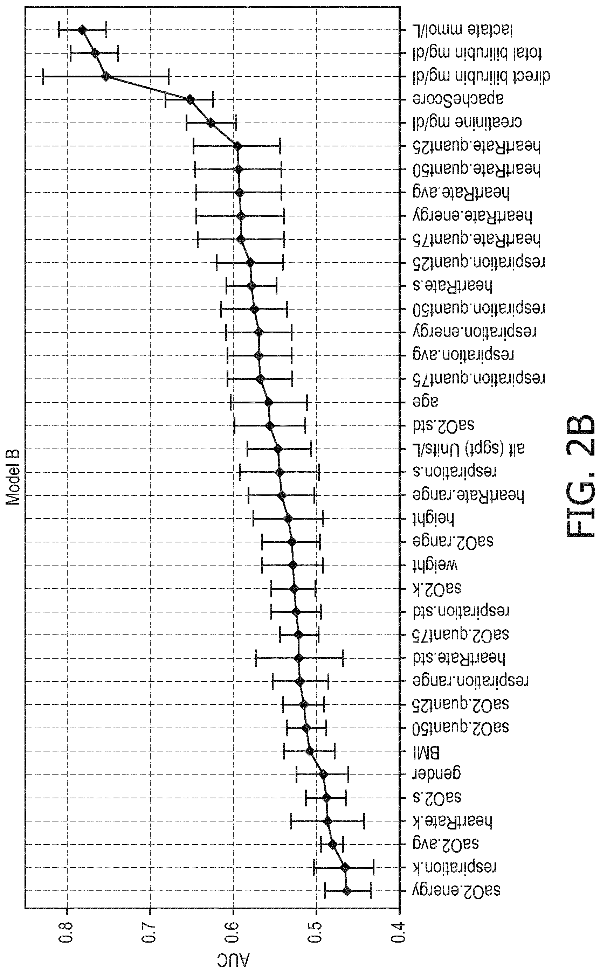

The discriminative power of different standalone factors (features) and their combinations were evaluated in order to define the optimal combination of features for DIC prediction. Such factors include biomarkers, features extracted from vital signs, subjects' characteristics and Apache score. Referring now to the figures, FIG. 1 illustrates initial statistical analysis showing a significant difference in biomarker concentration for the biomarkers bilirubin, lactate and creatinine, between populations that developed and those that did not develop DIC. FIG. 1 demonstrates the discriminative power of such biomarkers for DIC prediction. Similarly, the discriminative power of each considered feature was evaluated with a logistic classifier and expressed in terms of area under (AUC) the receiving operating characteristic (ROC). *See* FIGS. 2A, 2B.

The ROC curve illustrates the performance of a binary classifier for different operating points. The curve is drawn by plotting the true positive rate (TPR) against the false positive rate (FPR) calculated for the different operating points. The 'optimal cutoff' *(see* Table 5, *Opt th)* is defined in this document as the value which provides the highest achievable average or sum of the TPR (sensitivity) and specificity (1 - false positive rate FPR) and aims to give an indication of the sensitivity and specificity that the models are capable of. Note that the cut-off that is optimal in a real world sense depends on the specific application, and this invention does not aim to propose an optimal cut-off. A further (widely used) parameter extracted from the ROC curve and used for models comparison is the Area Under the Curve (AUC), which can be interpreted as the probability that the classifier will assign a higher score to a randomly chosen positive example than to a randomly chosen negative example.

FIGS. 2A and 2B are plots which show the area under the curve (AUC) for single biomarkers, subjects characteristics, Apache score and statistic features extracted from vital signs, for the 24-hours model (FIG. 2A) and the 72 ICU admittance hours model (FIG. 2B). Mean AUC value and associated standard deviation have been evaluated with 10-Fold cross validation on the training set. Acronyms of the used statistics are: std: standard deviation, avg: mean, k: kurtosis, s: skewness, quant25, quant50, quant75: quantile 0.25, 0.50, 0.75.

FIGS. 2A and 2B demonstrate the predicting power of the selected biomarkers, each of which obtained an AUC above 0.60.

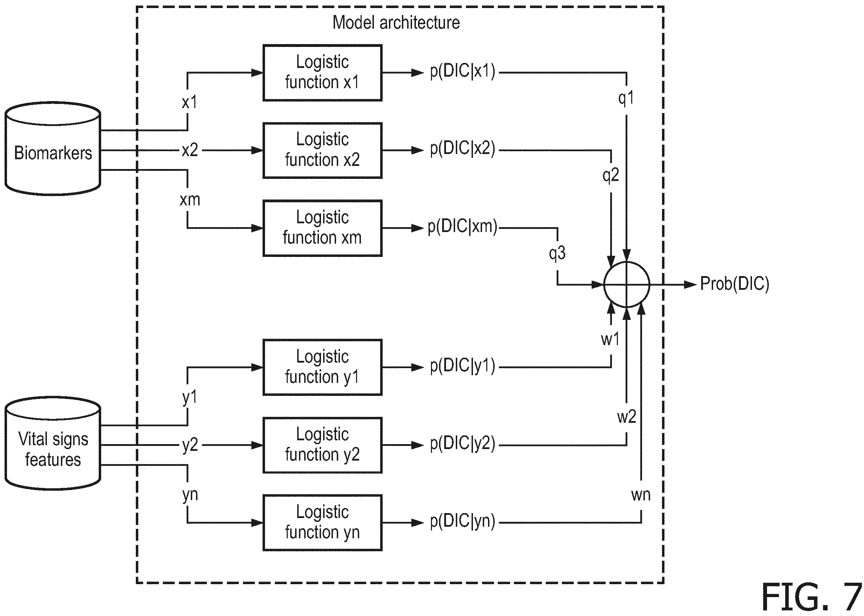

In order to select the optimal feature set, and therefore the best model architecture, a feature selection procedure was implemented. First, all features that had an AUC below 0.55 were excluded and *n* features were kept. Afterwards *n* single feature logistic regression models were trained (where feature is either vital sign extracted statistic or a biomarkers level, maximum biomarker level where multiple measurements are available). The DIC score was calculated by combining the output of the multiple univariate logistic models with a weighted average, with the weight being proportional to the univariate logistic model AUC calculated on the training set. The schema of this architecture is depicted in FIG. 7.

Considering the output of each logistic model $P(Y|X_i)$ as the likelihood of getting DIC given a predictor $X_i$ and $AUC_i$, the area under the ROC curve achieved by $X_i$ calculated on the training set, the weight assigned to each predictor was calculated as follows:

$$\alpha = 1/(\sum_i AUC_i)$$

$$w_i = AUC_i \times \alpha$$

The risk of getting DIC ($P(Y)$) is finally calculated as:

$$P(Y) = \sum_{i=1}^{n} P(Y|X_i) \times w_i$$

In order to select the optimal set of models (X, features) included in the ensemble classifier described above, the performance of different model combinations was evaluated. To reduce the number of combinations we have constrained the maximum number of items for combination to three. We have computed the best configuration using only vital signs, only biomarkers and their combination.

The combinations which provided the highest AUC for only biomarkers, only vital signs features and their combination have been selected and tested on the independent test set (see Table 4).

The results of the selection process and therefore the best model configurations are shown in Table 5.

Table 5 Performance of DIC prediction models evaluated on a test set

| | Opt th | Se % | Sp% | AUC | Nr. cases | Nr. Ctrls |
|---|---|---|---|---|---|---|
| **Biomarkers** | | | | | | |
| creatinine, lactate, total bilirubin* | 0.49 | 57.8 | 84.9 | **0.79** | 128 | 165 |
| creatinine, lactate, total bilirubin** | 0.52 | 75.0 | 79.3 | **0.82** | 180 | 159 |
| | | | | | | |
| **Vital signs features** | | | | | | |
| Standard deviation of saO2, quantile 0.25 of heartRate, Kurtosis of saO2* | 0.57 | 99.7 | 4.6 | **0.66** | 288 | 132 |
| Quantile 0.25 of heartRate, quantile 0.25 of respiration, Skewness of heartRate** | 0.51 | 91.0 | 18.2 | **0.62** | 631 | 439 |
| | | | | | | |
| **Laboratory tests and vital signs** | | | | | | |
| lactate, total bilirubin, energy of respiration* | 0.41 | 86.0 | 65.4 | **0.85** | 50 | 26 |
| lactate, total bilirubin, skewness of heartRate** | 0.57 | 70.5 | 83.9 | **0.83** | 166 | 155 |
| **Acronyms: standard deviation (std), kurtosis (k), quantile 0.25 (quant25), Oxygen saturation (saO2), optimal threshold calculated on the ROC for assigning DIC class (Opt th), sensitivity (Se), specificity (Sp), Nr. cases (number of patients included in test set that got DIC), Nr. Ctrls (number of patients included in test set that did not get DIC)** **\*Feature set for predicting DIC 24 hours in advance** **\*\*Feature set for predicting DIC within 72 hours from ICU admittance** | | | | | | |

The combination of bilirubin, lactate and creatinine was shown to be largely discriminative, achieving an AUC of 0.79 and 0.82 for the 24-hours model and 72 ICU admittance hours model, respectively. The lower accuracy obtained by the 24-hours model was probably related to the constraint of considering data collected at least 24 hours before DIC diagnosis. The assumption is that the closer to the diagnosis, the higher the increase and therefore the higher the predictability. According to literature the increase of such biomarkers is in most cases related to organ failure. The obtained results suggest that multiple organs are starting to fail, possibly due to lower perfusion caused by micro-vascular thromboses that could represent a first clinical sign of DIC occurrence. A further unexpected result of this work was the discovery that the coupling of vital signs with total bilirubin and lactate provides for a more accurate prediction. In fact, the largest accuracy was achieved by the combination of lactate, total bilirubin and respiration energy for the 24-hours model, which obtained an AUC curve of 0.85 and lactate, total bilirubin and heart rate skewness for 72 ICU admittance hours model, which obtained an AUC of 0.83.

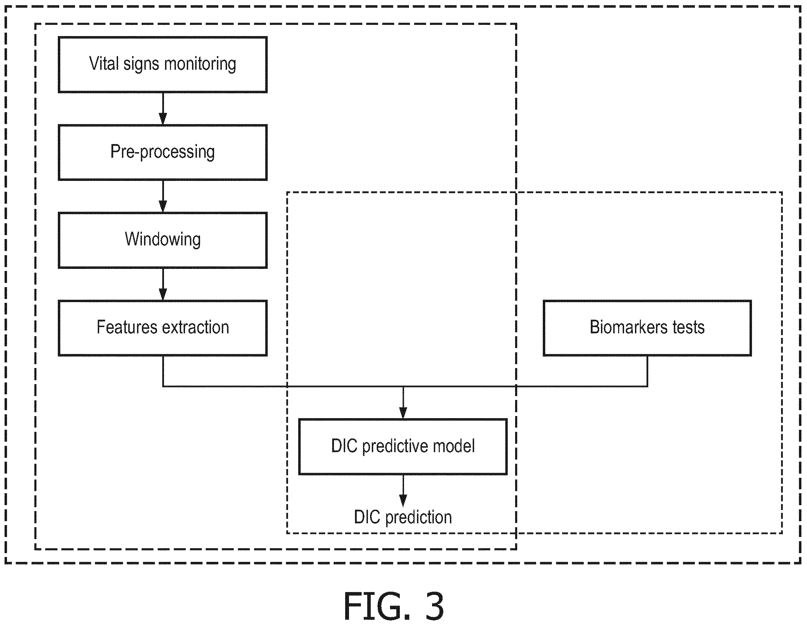

Given the results obtained by the feature selection, three possible implementations are indicated: a model based on biomarkers, a model based on vital signs features and a model composed by the combination of biomarkers and vital signs features. The description of the general DIC risk assessment framework and the three implementation versions are show in FIG. 3. The implementation version based on vital signs features (orange block in FIG. 3) requires:

(a) Vital signs monitoring: continuous signals recording;
(b) Pre-processing: outliers removal based on physiological range;
(c) Windowing: 2 hours signal segmentation with 1 hour overlap; and
(d) Feature extraction: calculation of statistical features from vital signs windows;

The implementation version based on biomarkers requires at least a one-time, but preferably regular, e.g. daily biomarkers tests (green block, FIG. 3). And the implementation version based on biomarkers and vital signs requires the combination of the two systems (red block, FIG. 3).

In the example shown, a logistic function is used to estimate the probability of DIC given a selected single feature; feature based probabilities are then combined in a weighted average to obtain the final DIC prediction score expressed in terms of probability. The weights depend on the predictive accuracy of the separate estimator 8. Each of the algorithms (*i.e.* logistic function and weighted averaging) may be replaced by other methods known in the field (*e.g.,* artificial neural networks, decision trees, *etc.*). Alternatively, the scheme below may be treated as a Bayesian network with probability tables estimated from the train data (naive Bayes) or from *a priori* knowledge (if available). Different blocks of the scheme could be combined in different ways and independently used to assess DIC risk probability.

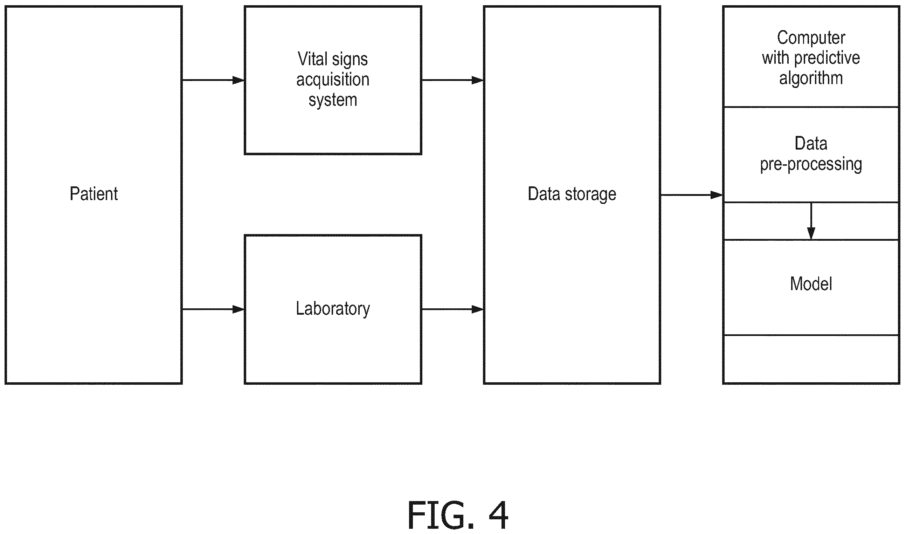

The description of the general DIC risk assessment system is shown in FIG. 4. Such system includes:

(a) A system for vital signs acquisition
(b) A laboratory to measure biomarkers concentration
(c) Data storage system such as a local drive
(e) A computer which hosts the algorithm consisting of a block for data pre-processing *(see*

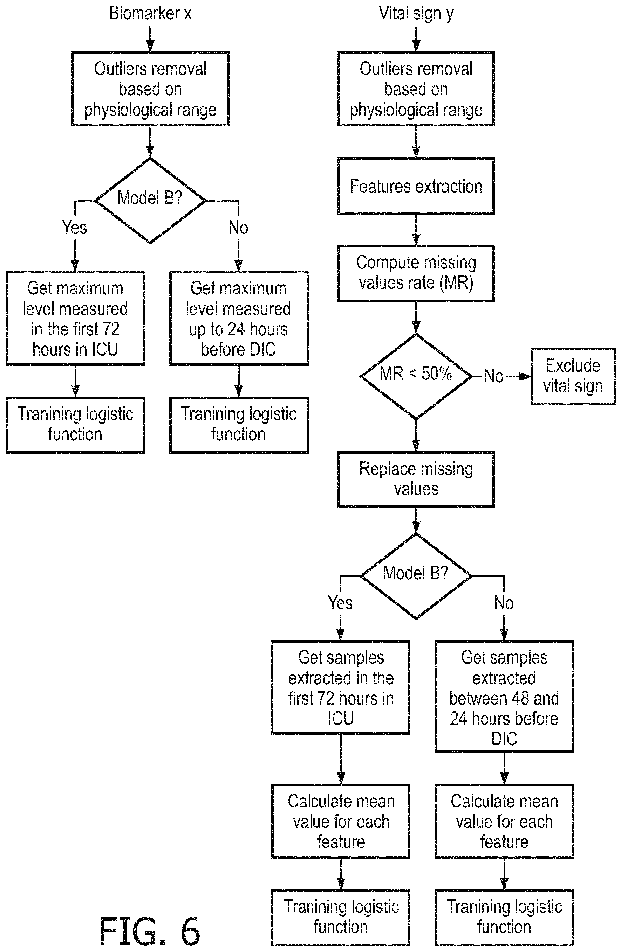

FIG. 6), and a block with the predictive model *(see* FIG. 7).

The output of the system of FIG. 4 could be used as input for a decision-making system responsible of deciding whether an intervention is needed and, in positive case, which kind of intervention would be more beneficial for the patient. A typical intervention could be a drug therapy (*e.g.* anti-coagulant, anti-arrhythmic).

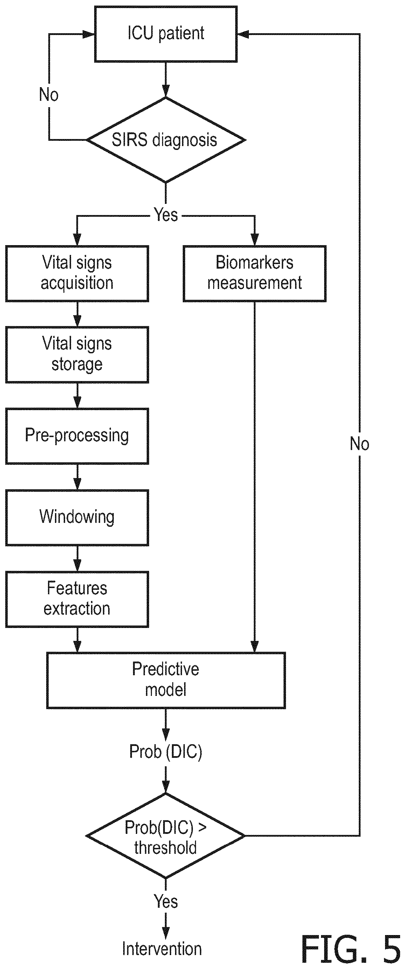

FIG. 5 shows a flowchart of the process that leads from the patient diagnosis to the DIC prediction. Particularly, FIG. 5 is a description of the process from the admission of the patient in the ICU to the risk assessment for DIC. The pre-processing steps that precede the training of the logistic functions included in the final predictive model are explained in detail in FIG. 6, while FIG. 7 shows the architecture of the proposed predictive model.

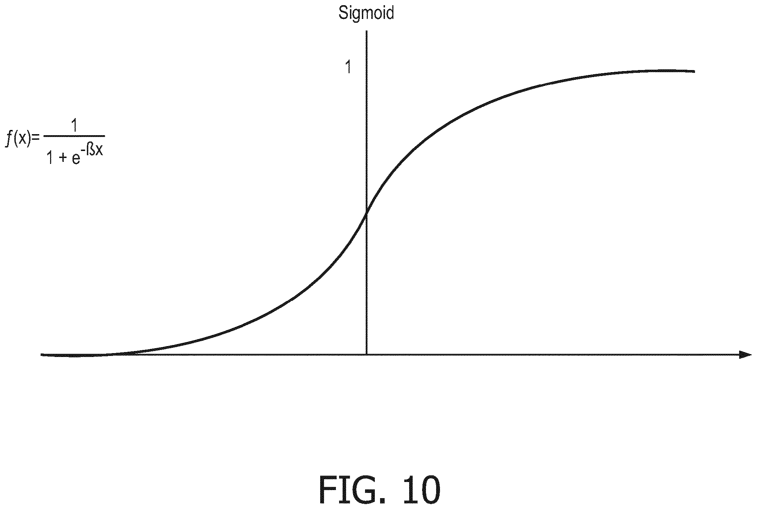

In the current application example, the scheme of the implemented model is shown in FIG. 7 (for which the results are shown). A logistic function is used to estimate the probability of DIC given a selected single feature *(see* FIG. 10). Feature based probabilities are then combined with weighted average to obtain the final DIC prediction score.

The weights depend on the predictive accuracy of the separate estimators calculated over the training set. Note that each of the algorithms may be replaced by other machine learning methods known in the field (*e.g.,* artificial neural networks, decision trees, *etc.*). Alternatively, the scheme below may be treated as a Bayesian network with probability tables estimated from the train data (naive Bayes) or from *a priori* knowledge (if available). Different blocks of the scheme could be combined in different ways and independently used to assess DIC risk probability.

FIG. 9A and 9B illustrate receiver operating curves calculated for 24-hours model and 72-hours ICU admittance model based on: laboratory tests only, vital signs only, combination of laboratory tests and vital signs. The AUC calculated for each curve is reported in Table 5.

**Claims**

1. A computer program product, comprising a computer-readable code to be executed by one or more processors when retrieved from a non-transitory computer-readable medium, the computer-readable program code including instructions to carry out a method for assessing the risk of the development of disseminated intravascular coagulation in a patient diagnosed with systematic inflammatory response syndrome by means of a computing device with a graphical user interface, the method comprising:

   controlling a means for acquiring and storing patient specific diagnostic data to acquire said patient specific diagnostic data, including initial vital signs and biomarker measurement data from said patient, and to input and store said patient specific diagnostic data on a non-transitory computer readable storage medium, wherein the biomarker measurement data comprises total bilirubin and lactate measurement data;
   controlling said means for acquiring and storing patient specific diagnostic data to monitor said vital signs of said patient and to continuously input and store said vital signs and biomarker measurement data on said non-transitory computer readable storage medium;
   pre-processing said vital sign data, including discarding samples which are outside a physiological range and removing outliers from said data;
   windowing of said vital signs data to obtain vital signs windows;
   calculating statistical features from said vital signs windows;
   analyzing said statistical features in combination with the biomarker measurement data by inputting the statistical features and the biomarker measurements into a predictive model that is stored on said non-transitory computer readable storage medium, wherein the predictive model is configured to output a disseminated intravascular coagulation prediction; and
   determining whether the disseminated intravascular coagulation prediction meets a disseminated intravascular coagulation probability threshold, wherein said probability threshold is predictive of the likely development of disseminated intravascular coagulation.

2. The computer program product of claim 1, wherein the biomarker measurement data further comprises creatinine.

3. The computer program product of claim 1, wherein the vital signs include at least respiration.

4. The computer program product of claim 3, wherein calculating the statistical features for respiration windows comprises calculating the energy of respiration.

5. The computer program product of claim 1, wherein the vital signs include at least heart rate.

6. The computer program product of claim 5, wherein calculating the statistical features for heart rate windows comprises calculating the skewness of the heart rate.

7. The computer program product of claim 1, wherein the predictive model comprises a logistic function, wherein the statistical features and the biomarker measurement data are combined in a weighted average to obtain the disseminated intravascular coagulation prediction.

8. The computer program product of claim 1, wherein said biomarker measurement data is analyzed with respect to said predictive model that is stored on said non-transitory computer readable storage medium.

9. The computer program product of claim 1, wherein said vital sign data and said biomarker measurement data are both analyzed together with respect to said predictive model that is stored on said non-transitory computer readable storage medium.

10. A non-transitory computer readable storage medium tangibly encoded with computer-executable instructions of the computer program product of any one of claims 1 to 9.

11. A system comprising:

   a non-transitory computer readable storage medium for storing patient specific diagnostic data;
   a means for acquiring patient specific diagnostic data and storing said patient specific diagnostic data on said non-transitory computer readable storage medium; and

a computing device having a graphical user interface and a processor programmed with the computer program product of any one of claims 1 to 9.

**Patentansprüche**

1. Computerprogrammprodukt, das einen computerlesbaren Code umfasst, der von einem oder mehreren Prozessoren ausgeführt werden soll, wenn er von einem nicht flüchtigen computerlesbaren Medium abgerufen wird, wobei der computerlesbare Programmcode Anweisungen zum Ausführen eines Verfahrens zum Bewerten des Risikos der Entwicklung einer disseminierten intravaskulären Gerinnung bei einem Patient mit der Diagnose eines systematischen Entzündungsreaktionssyndroms mittels einer Rechenvorrichtung mit einer grafischen Benutzeroberfläche einschließt, wobei das Verfahren Folgendes umfasst:

   Steuern eines Mittels zum Erfassen und Speichern patientenspezifischer Diagnosedaten, um die patientenspezifischen Diagnosedaten, einschließlich anfänglicher Vitalzeichen und Biomarker-Messdaten, von dem Patienten zu erfassen und um die patientenspezifischen Diagnosedaten in ein nicht flüchtiges, computerlesbares Speichermedium einzugeben und auf diesem zu speichern, wobei die Biomarker-Messdaten Gesamtbilirubin- und Laktat-Messdaten umfassen;
   Steuern der Mittel zum Erfassen und Speichern patientenspezifischer Diagnosedaten, um die Vitalzeichen des Patienten zu überwachen und um die Vitalzeichen und Biomarker-Messdaten kontinuierlich in das nicht flüchtige, computerlesbare Speichermedium einzugeben und auf diesem zu speichern;
   Vorverarbeiten der Vitalzeichendaten, einschließlich eines Verwerfens von Proben, die außerhalb eines physiologischen Bereichs liegen, und eines Entfernens von Ausreißern aus den Daten;
   Fensterung der Vitalzeichendaten, um Vitalzeichenfenster zu erhalten;
   Berechnen statistischer Merkmale aus den Vitalzeichenfenstern;
   Analysieren der statistischen Merkmale in Kombination mit den Biomarker-Messdaten durch Eingeben der statistischen Merkmale und der Biomarker-Messungen in ein Vorhersagemodell, das auf dem nicht flüchtigen computerlesbaren Speichermedium gespeichert ist, wobei das Vorhersagemodell so konfiguriert ist, dass es eine Vorhersage der disseminierten intravaskulären Gerinnung ausgibt; und
   Bestimmen, ob die Vorhersage der disseminierten intravaskulären Gerinnung einen Wahrscheinlichkeitsschwellenwert für die disseminierte intravaskuläre Gerinnung erreicht, wobei der Wahrscheinlichkeitsschwellenwert die wahrscheinliche Entwicklung einer disseminierten intravaskulären Gerinnung vorhersagt.

2. Computerprogrammprodukt nach Anspruch 1, wobei die Biomarker-Messdaten weiter Kreatinin umfassen.

3. Computerprogrammprodukt nach Anspruch 1, wobei die Vitalzeichen zumindest Atmung einschließen.

4. Computerprogrammprodukt nach Anspruch 3, wobei das Berechnen der statistischen Merkmale für Atmungsfenster das Berechnen der Atmungsenergie umfasst.

5. Computerprogrammprodukt nach Anspruch 1, wobei die Vitalzeichen zumindest die Herzfrequenz einschließen.

6. Computerprogrammprodukt nach Anspruch 5, wobei das Berechnen der statistischen Merkmale für Herzfrequenzfenster das Berechnen der Asymmetrie der Herzfrequenz umfasst.

7. Computerprogrammprodukt nach Anspruch 1, wobei das Vorhersagemodell eine logistische Funktion umfasst, wobei die statistischen Merkmale und die Biomarker-Messdaten in einem gewichteten Durchschnitt kombiniert werden, um die Vorhersage der disseminierten intravaskulären Gerinnung zu erhalten.

8. Computerprogrammprodukt nach Anspruch 1, wobei die Biomarker-Messdaten in Bezug auf das auf dem nicht flüchtigen, computerlesbaren Speichermedium gespeicherte Vorhersagemodell analysiert werden.

9. Computerprogrammprodukt nach Anspruch 1, wobei die Vitalzeichendaten und die Biomarker-Messdaten beide gemeinsam in Bezug auf das auf dem nicht flüchtigen, computerlesbaren Speichermedium gespeicherte Vorhersagemodell analysiert werden.

10. Nicht flüchtiges, computerlesbares Speichermedium, das materiell mit computerausführbaren Anweisungen des Computerprogrammprodukts nach einem der Ansprüche 1 bis 9 codiert ist.

**11.** System, umfassend:

ein nicht flüchtiges, computerlesbares Speichermedium zum Speichern patientenspezifischer Diagnosedaten;
ein Mittel zum Erfassen patientenspezifischer Diagnosedaten und zum Speichern der patientenspezifischen Diagnosedaten auf dem nicht flüchtigen computerlesbaren Speichermedium; und
eine Rechenvorrichtung, die eine grafische Benutzeroberfläche und einen Prozessor aufweist, der mit dem Computerprogrammprodukt nach einem der Ansprüche 1 bis 9 programmiert ist.

**Revendications**

**1.** Produit de programme informatique, comprenant un code lisible par ordinateur à exécuter par un ou plusieurs processeurs lorsqu'il est extrait d'un support non transitoire lisible par ordinateur, le code de programme lisible par ordinateur incluant des instructions pour mettre en œuvre un procédé pour évaluer le risque de développement d'une coagulation intravasculaire disséminée chez un patient auquel on a diagnostiqué un syndrome de réponse inflammatoire systématique au moyen d'un dispositif informatique doté d'une interface utilisateur graphique, le procédé comprenant :

la commande d'un moyen pour acquérir et stocker des données de diagnostic spécifiques au patient pour acquérir lesdites données de diagnostic spécifiques au patient, incluant des données initiales de signes vitaux et de mesure de biomarqueurs dudit patient, et pour saisir et stocker lesdites données de diagnostic spécifiques au patient sur un support de stockage non transitoire lisible par ordinateur, dans lequel les données de mesure de biomarqueurs comprennent des données de mesure de bilirubine totale et de lactate ;
la commande desdits moyens pour acquérir et stocker des données de diagnostic spécifiques au patient pour surveiller lesdits signes vitaux dudit patient et pour saisir et stocker en continu lesdites données de mesure de biomarqueurs et de signes vitaux sur ledit support de stockage non transitoire lisible par ordinateur ;
le prétraitement desdites données de signes vitaux, incluant l'élimination d'échantillons qui sont en dehors d'une plage physiologique et la suppression de valeurs aberrantes desdites données ;
le fenêtrage desdites données de signes vitaux pour obtenir des fenêtres de signes vitaux ;
le calcul de caractéristiques statistiques à partir desdites fenêtres de signes vitaux ;
l'analyse desdites caractéristiques statistiques en combinaison avec les données de mesure de biomarqueurs en saisissant les caractéristiques statistiques et les mesures de biomarqueurs dans un modèle prédictif qui est stocké sur ledit support de stockage non transitoire lisible par ordinateur, dans lequel le modèle prédictif est configuré pour délivrer une prédiction de coagulation intravasculaire disséminée ; et
la détermination permettant de savoir si la prédiction de coagulation intravasculaire disséminée répond à un seuil de probabilité de coagulation intravasculaire disséminée, dans lequel ledit seuil de probabilité est annonciateur du développement probable d'une coagulation intravasculaire disséminée.

**2.** Produit de programme informatique selon la revendication 1, dans lequel les données de mesure de biomarqueurs comprennent en outre la créatinine.

**3.** Produit de programme informatique selon la revendication 1, dans lequel les signes vitaux incluent au moins la respiration.

**4.** Produit de programme informatique selon la revendication 3, dans lequel le calcul des caractéristiques statistiques pour des fenêtres de respiration comprend le calcul de l'énergie de respiration.

**5.** Produit de programme informatique selon la revendication 1, dans lequel les signes vitaux incluent au moins la fréquence cardiaque.

**6.** Produit de programme informatique selon la revendication 5, dans lequel le calcul des caractéristiques statistiques pour des fenêtres de fréquence cardiaque comprend le calcul de l'asymétrie de la fréquence cardiaque.

**7.** Produit de programme informatique selon la revendication 1, dans lequel le modèle prédictif comprend une fonction logistique, dans lequel les caractéristiques statistiques et les données de mesure de biomarqueurs sont combinées en une moyenne pondérée pour obtenir la prédiction de coagulation intravasculaire disséminée.

**8.** Produit de programme informatique selon la revendication 1, dans lequel lesdites données de mesure de biomarqueurs sont analysées par rapport audit modèle prédictif qui est stocké sur ledit support de stockage non transitoire lisible par ordinateur.

9. Produit de programme informatique selon la revendication 1, dans lequel lesdites données de signes vitaux et lesdites données de mesure de biomarqueurs sont toutes deux analysées ensemble par rapport audit modèle prédictif qui est stocké sur ledit support de stockage non transitoire lisible par ordinateur.

10. Support de stockage non transitoire lisible par ordinateur codé de manière tangible avec des instructions exécutables par ordinateur du produit de programme informatique selon l'une quelconque des revendications 1 à 9.

11. Système, comprenant :

un support de stockage non transitoire lisible par ordinateur pour stocker des données de diagnostic spécifiques au patient ;
un moyen pour acquérir des données de diagnostic spécifiques au patient et stocker lesdites données de diagnostic spécifiques au patient sur ledit support de stockage non transitoire lisible par ordinateur ; et
un dispositif informatique présentant une interface utilisateur graphique et **un** processeur programmé avec le produit de programme informatique selon l'une quelconque des revendications 1 à 9.

FIG. 1

FIG. 2A

FIG. 2B

FIG. 3

FIG. 4

EP 3 639 276 B1

FIG. 5

FIG. 6

FIG. 7

EP 3 639 276 B1

EP 3 639 276 B1

Prediction

k

Biomarker B → | Apply the logistic function: f(x)= 1/(1-e^(-kx)) | → P(DIC|x)

Pre-processing          Windowing          Features extraction                          Prediction

k

Vital sign V → | Remove values outside valid range | → | Divide V in windows 120 mins long, 60 mins overlap | → | Calculate the statistic s from each window | → | Calculate the feature y as the mean of the statistic s calculated over all the windows | → | Apply the logistic function f(y) = 1/(1-e^(-my)) | → P(DIC|y)

# FIG. 8

FIG. 9A

FIG. 9B

EP 3 639 276 B1

$$f(x) = \frac{1}{1 + e^{-\beta x}}$$

Sigmoid

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009123737 A

**Non-patent literature cited in the description**

- **DI NISIO, M. et al.** Diagnosis and treatment of disseminated intravascular coagulation: guidelines of the Italian Society for Haemostasis and Thrombosis (SISET). *Thrombosis research*, 2012, vol. 129, e177-e184
- **LEVI. M. et al.** Disseminated intravascular coagulation. *New England Journal of Medicine*, 1999, vol. 341, 586-592

- **KINASEWITZ, G.T. et al.** Prognostic value of a simple evolving disseminated intravascular coagulation score in patients with severe sepsis. *Critical Care Medicine*, 2005, vol. 33, 2214-2221
- Criteria for diagnosis of DIC based on the analysis of clinical and laboratory findings in 345 DIC patients collected by the Research Committee on DIC in Japan. **KOBAYASHI, N. et al.** Disseminated Intravascular Coagulation. Karger Publishers, 1983, 265-275